(19) Europäisches Patentamt / European Patent Office / Office européen des brevets

(11) **EP 2 069 099 B1**

(12) **EUROPÄISCHE PATENTSCHRIFT**

(45) Veröffentlichungstag und Bekanntmachung des Hinweises auf die Patenterteilung:
**20.07.2011 Patentblatt 2011/29**

(21) Anmeldenummer: **07802266.2**

(22) Anmeldetag: **11.09.2007**

(51) Int Cl.:
*B23K 26/04* *(2006.01)*      *A61F 9/009* *(2006.01)*
*A61F 9/01* *(2006.01)*

(86) Internationale Anmeldenummer:
**PCT/EP2007/007914**

(87) Internationale Veröffentlichungsnummer:
**WO 2008/040436 (10.04.2008 Gazette 2008/15)**

(54) **VORRICHTUNG UND VERFAHREN ZUR MATERIALVERARBEITUNG UNTER VERWENDUNG EINES TRANSPARENTEN KONTAKTELEMENTS**

DEVICE AND METHOD FOR PROCESSING MATERIAL USING A TRANSPARENT CONTACT ELEMENT

DISPOSITIF ET PROCEDE DE TRAITEMENT DE MATERIAUX PAR RECOURS A UN ELEMENT TRANSPARENT DE CONTACT

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **29.09.2006 DE 102006046370**

(43) Veröffentlichungstag der Anmeldung:
**17.06.2009 Patentblatt 2009/25**

(73) Patentinhaber: **Carl Zeiss Meditec AG**
**07745 Jena (DE)**

(72) Erfinder:
• **BISCHOFF, Mark**
**07749 Jena (DE)**
• **STOBRAWA, Gregor**
**07743 Jena (DE)**

(74) Vertreter: **Geyer, Fehners & Partner**
**Patentanwälte**
**Perhamerstrasse 31**
**80687 München (DE)**

(56) Entgegenhaltungen:
| EP-A- 1 159 986 | WO-A-03/002008 |
| WO-A-2004/032810 | WO-A-2005/039462 |
| DE-A1- 10 354 025 | DE-A1- 19 831 674 |
| US-A- 5 549 632 | US-B1- 6 373 571 |

EP 2 069 099 B1

**Beschreibung**

Die Erfindung bezieht sich auf ein Verfahren zum Vorbereiten einer Vorrichtung zur Materialbearbeitung durch Erzeugung optischer Durchbrüche in oder an einem Objekt, die eine variable, dreidimensional wirkende Fokusversielleinrichtung zur Fokussierung gepulster Bearbeitungslaserstrahlung auf verschiedene Orte im oder auf dem Objekt aufweist, wobei an der Vorrichtung ein für die Bearbeitungslaserstrahlung transparentes auf das Objekt aufzusetzendes Kontaktelement befestigt wird, das auf seiner auf das Objekt aufzusetzenden Seite eine Kontakt-Fläche und eine dieser gegenüberliegende Eintritts-Fläche für die Bearbeitungslaserstrahlung aufweist, die jeweils vorbekannte Form haben, vor der Bearbeitung des Objektes die Lage der Eintritts- oder der Kontakt-Fläche bezüglich der Fokusverstelleinrichtung mittels Einstrahlung von Laserstrahlung auf die Fläche bestimmt wird. indem Meßlaserstrahlung mittels der variablen Fokusverstelleinrichtung nahe der oder auf die Fläche fokussiert wird, wobei die Energiedichte der fokussierten Meßlaserstfahlung zur Erzeugung eines optischen Durchbruches zu gering ist, und die Fokuslage der Meßlaserstrahlung in einer Meßfläche verstellt wird, die die erwartete Lage der Fläche schneidet.

Die Erfindung bezieht sich weiter auf eine Materialbearbeitungs-Vorrichtung mit, einem Bearbeitungslaser, der gepulste Beatbeitungstaserstrahlung bereitstellt, einer Optikeinrichtung zum Fokussieren der Bearbeitungslaserstrahlung in oder auf ein zu bearbeitendes Objekt derart, daß im Fokus optische Durchbrüche entstehen, einer Fokusverstelleinrichtung zum variablen Verstellen der Fokuslage im oder auf dem Objekt, einem an der Vorrichtung befestigbares Kontaktelement zum Aufsetzen auf das Objekt, das eine auf das Objekt aufzusetzende Kontakt-Fläche und eine dieser gegenüberliegende Eintritts-Fläche für die Bearbeitungslaserstrahlung aufweist, die jeweils vorbekannte Form haben, und einer Steuereinrichtung zur Bestimmung der Lage der Einritts- oder der Kontakt-Fläche nach der Befestigung des Kontaktelementes und vor der Bearbeitung des Objektes, die den Bearbeitungslaser und die Fokusverstelleinrichtung ansteuert, wobei eine ebenfalls von der Steuereinrichtung, angesteuerte Meßlaserstrahtungsquelle zur Abgabe von Meßlaserstrahlung vorgesehen ist, deren Meßlaserstrahlung die Fokusverstelleinrichtung und die Optikeinrichtung durchläuft und im Fokus keine optischen Durchbrüche bewirkt, wobei die Steuereinrichtung zur Bestimmung der Lage der Fläche den Fokus der Meßlaserstrahlung in einer Meßfläche verstellt, welche die zu erwartende Lage der Fläche schneidel.

Bei der Materialbearbeitung wird oft eine Laserbearbeitungsvorrichtung zum Abrastern der zu bearbeitenden Gebiete des Objektes mit einem Bearbeitungslaserstrahl eingesetzt. Die Genauigkeit der Positionierung des Laserstrahls bestimmt dabei in der Regel die bei der Bearbeitung erzielte Präzision. Wird der Laserstrahl, in ein Bearbeitungsvolumen fokussiert, bedarf es einer exakten dreidimensionalen Positionierung. Für eine hochgenaue Bearbeitung ist es deshalb in der Regel unerläßlich, das Objekt in exakt definierter Lage zur Laserbearbeitungsvorrichtung zu halten. Für solche Anwendungen dient das eingangs genannte Kontaktelement, da mit ihm das zu bearbeitende Objekt fixiert werden kann, wodurch definierte Verhältnisse bis zum Bearbeitungsvolumen erreichbar sind. Das Kontaktelement wird damit Teil des Strahlenganges der Bearbeitungslaserstrahlung.

Dies ist insbesondere bei der Mikrobearbeitung von Materialien notwendig, die nur eine geringe lineare optische Absorption im Spektralbereich der bearbeitenden Laserstrahlung aufweisen. Bei solchen Materialien werden üblicherweise nicht-lineare Wechselwirkungen zwischen Laserstrahlung und Material ausgenutzt, meist in Form eines optischen Durchbruches, der im Fokus hochenergetischer Laserstrahlung erzeugt wird. Da die bearbeitende Wirkung dann nur im Laserstrahlfokus stattfindet, ist es unerläßlich, die Lage des Fokus exakt dreidimensional auszurichten. Zusätzlich zu einer zweidimensionalen Ablenkung des Laserstrahls ist somit eine exakte Tiefeneinstellung der Fokuslage erforderlich. Das Kontaktelement dient dazu, konstante und auch mit einer gewissen Genauigkeit bekannte optische Verhältnisse im Strahlengang zum Objekt sicherzustellen, indem er Objekt und Laserbearbeitungsvorrichtung mechanisch koppelt und zudem der Objektoberfläche eine Form mit bekannter optischer Wirkung verleiht.

Eine typische Anwendung für ein solches Kontaktglas ist das als Femtosekunden-LASIK bekannte augenoptische Operationsverfahren, bei dem die als Therapiegerät ausgebildete Laserbearbeitungsvorrichtung einen Laserstrahl in die Hornhaut auf einen Fokus in der Größenordnung eines Mikrometers fokussiert. Im Fokus entsteht dann ein Plasma, das eine lokale Trennung des Hornhautgewebes bewirkt. Durch geeignete Aneinanderreihung der auf diese Weise erzeugten lokalen Trennungszonen werden mikroskopische Schnitte realisiert, z.B. ein bestimmtes Hornhautteilvolumen isoliert.

Die Lage des Kontaktelementes ist für dieses Verfahren genauigkeitsbeslimmend und deshalb in der Literatur vielfältig hinsichtlich der Lagebestimmung behandelt:

Aus der US 6.373.571 ist eine mit Referenzmarken versehene Kontaktlinse bekannt. Diese Kontaktlinse wird mittels einer separaten Meßvorrichtung einjustiert, wodurch ein relativ aufwendiger Aufbau bedingt ist. Ein weiteres Beispiel für ein Kontaktelement ist in der EP 1 159 986 A2 beschrieben. Es ähnelt der Kontaktlinse der US 6.373.571, weist aber zusätzlich einen Rand in Form einer Halterung mit Strichmarken auf, die dem Chirurgen eine visuelle Ausrichtung ermöglichen. Diese ist jedoch in der Regel zu unpräzise.

Da das Kontaktelement üblicherweise Kontakt mit dem zu bearbeitenden Objekt hat, ist es meist erforderlich, für jedes Objekt einen eigenen neuen Adapter einzusetzen. Dies gilt besonders unter dem Gesichtspunkt der Sterilität bei augenchirurgischen Verfahren. Daraus folgt, daß das Kontaktelement jedesmal vor einer Bearbeitung bzw. vor einem chirurgischen Eingriff an der Laserbearbeitungsvorrichtung, die dann z.B. als Therapiegerät ausgebildet ist, befestigt werden muß. Zur Befestigung ist es aus der WO 03/002008 A1 bekannt, das Kontaktglas in einer zangenartigen Einrichtung zu halten, die an der Laserbearbeitungsvorrichtung verriegelt ist. Die Verriegelung erfolgt über einen in einer Schiene geführten Kragen. Der Adapter wird quer zur optischen Achse formschlüssig eingeschoben. In der DE 19831674 A1 ist die Verwendung eines mechanischen Kopplungsmechanismus beschrieben, bei dem ein in schrägem Winkel an einer Fassung eines Kontaktglases befestigter Metallstab mittels eines Magneten oder Elektromagneten in einer Hülse gehalten wird: Diese Befestigungen legen aber die Lage des Kontaktelementes nicht ausreichend genau fest.

Aus der WO 05/039462 A1 ist es weiter bekannt, ein Kontaktglas mit Lage-Marken zu versehen und die Laserbehandlungsvorrichtung um eine konfokale Detektoreinheit zu erweitern, die im Zusammenhang mit eingestrahlter Meßlaserstrahlung erlaubt, die Lage der Marken zu erfassen und daraus die Position des Kontaktglases zu ermitteln. Die Genauigkeit, mit der die Lage des Kontaktglases bekannt ist, ist damit besser als die Genauigkeit, die durch den Befestigungsmechanismus, wie er beispielsweise in der DE 10354025 A1 beschrieben ist, und den Fertigungstoleranzen des Kontaktglases gegeben ist.

Die WO 04/032810 A2, die als bester Stand der Technik gesehen wird, verfolgt ebenfalls das Ziel, die Lage des Kontaktglases genau zu bestimmen. Sie beschreibt ein Verfahren bzw. eine Vorrichtung der eingangs beschriebenen Art. Um die Lage der Kontaktfläche des Kontaktglases, die auf das Auge gepreßt wird, exakt zu ermitteln, schlägt die Veröffentlichung vor, die Wirkung des Behandlungslasers auf die Kontaktfläche auszunutzen. Der Behandlungslaser wird so engesteuert, daß er an einer Vielzahl von Punkten fokussiert wird und Behandlungslaserstrahlungspulse abgibt. Diejenigen Laserstrahlpulse, die auf die Grenzfläche des Kontaktglases fokussiert werden, erzeugen durch nicht-lineare Wirkung einen optischen Durchbruch, der sich in einem entsprechenden Plasma-Funken äußert. Die Detektion eines Funkens erlaubt damit die Aussage, daß an der gegenwärtigen Fokusposition des Behandlungslaserstrahls die Grenzfläche liegt. Die Ermittlung einer ausreichenden Anzahl solcher Punkte, die beispielsweise in einer Ebene senkrecht zur optischen Achse des Behandlungslaserstrahls angeordnet werden können, wird dann dazu verwendet, die Lage des Kontaktglases zu ermitteln. In einem alternativen Ansatz schlägt die Druckschrift vor, nicht-lineare Effekte am Kontaktglas auszunutzen, die bei geringeren Energien der Behandlungslaserstrahlpulse entstehen, also bei Energien, bei denen kein optischer Durchbruch bewirkt wird. Solche nicht-linearen Effekte unterhalb der Energieschwelle für optische Durchbrüche treten natürlich nur bei bestimmten Kontaktelementmaterialien auf. Die Druckschrift erwähnt nicht-lineare Effekte in Form der zweiten Harmonischen der eingestrahlten Behandlungslaserstrahlung oder Weißlichtstrahlung entstehen. Oie Messung bzw. Erfassung solcher Strahlung stellt hohe chromatische Anforderungen, da Strahlung z.B. der doppelten der eingestrahlten Frequenz detektiert werden muß. Das optische System wird dadurch aufwendig.

In einem dritten Ansatz schlägt die genannte Veröffentlichung vor, die Lage der Kontaktfläche des Kontaktglases mittels einer Interferenzanordnung zu erfassen. Die dabei entstehenden Interferenzmuster sind jedoch allgemein für ungeübte Benutzer nicht zur Justierung bzw. Berücksichtigung tauglich. Automatisch können sie nur extrem schwer ausgewertet werden. Das Konzept der Entgegenhaltung, soweit es anwendungstauglich ist, erfordert entweder Einstrahlung hochenergetischer Laserstrahlung, die optische Durchbrüche an der Grenzfläche des Kontaktglases erzeugt, was unter Strahlenschulzgesichtspunkten nachteilig ist, oder ist auf Kontaktglasmaterialien angewiesen, die einen nicht-linearen Effekt für die Bearbeitungslaserstrahlung zeigen.

Bei Kontaktelementen ist üblicherweise die auf das Objekt aufzusetzende Kontaktfläche hochpräzise gefertigt. Die genannten Verfahren und Vorrichtungen der WO04/0328810 A2 ermitteln deshalb die Lage der Kontaktfläche, die z.B. plan sein kann.

Der Erfindung liegt die Aufgabe zugrunde, ein Verfahren bzw. eine Vorrichtung der eingangs genannten Art hinsichtlich der Bestimmung der Lage des Kontaktglases zu verbessern.

Diese Aufgabe wird erfindungsgemäß durch eine Verfahren gemäß Anspruch 1 gelöst.

Die Aufgabe wird weiter gelöst durch eine Vorrichtung der eingangs genannten Art, bei der eine konfokale Detektoreinrichtung vorgesehen ist, die aus dem Fokus der Meßlaserstrahlung rückgestreute oder reflektierte Strahlung konfokal detektiert und Meßsignale an die Steuereinrichtung liefert, und die Steuereinrichtung aus den Meßsignalen die Lage von Schnittpunkten zwischen Meßfläche und Eintritts- oder Kontakt-Fläche ermittelt, wobei die Steuereinrichtung nötigenfalls die Meßfläche variiert, insbesondere verschiebt, 4alls keine oder zu wenige Schnittpunkte auftreten, und die aus der Lage der Schnittpunkte und aus der vorbekannten Form der Eintritts oder Kontakt-Fläche deren Lage bestimmt.

Für die Lagebestimmung kommt natürlich die Kontaktfläche in frage. Ist die Geometrie des Kontaktelementes zwischen Eintrittsfläche und Kontaktfläche hinreichend präzise vorgegeben, kommt natürlich auch die Bestimmung der Lage der Eintrittsfläche in Betracht. Insbesondere bei planen oder kugelschaligen Kontaktelementen ist die Geometrie zwischen Eintritts- und Kontaktfläche i.d.R. so genau bei der Herstellung einstellbar, daß für solche Kontaktelemente auch die Eintrittsflächenlage ermittelt werden kann. Dies hat den Vorteil, daß ein deutlicher Brechzahlsprung an der üblicherweise nicht kontaktierten Eintrittsfläche besteht, unabhängig davon, ob das Kontaktelement schon auf das Objekt aufgesetzt ist.

Die Erfindung verwendet zur Detektion also eine lineare Wechselwirkung zwischen Meßstrahlung und der zu erfassenden Fläche (also Eintritts- oder Kontaktfläche). Es muß weder Laserstrahlung auf eine Energiedichte fokussiert werden, die einen optischen Durchbruch erreicht, noch muß das Material des Kontaktglases eine nicht-lineare Wechselwirkung mit der Meßlaserstrahlung unterhalb der Energieschwelle für einen optischen Durchbruch zeigen. Zudem muß die verwendete Optik (z.B. ein Objektiv) nur Strahlung der gleichen Wellenlänge wie die Behandlungsstrahlung aufnehmen, was keine neuen (chromatischen) Anforderungen aufwirft. Der Aufbau wird durch die Detektionsaufgabe also, anders als beim Stand der Technik, nicht komplexer und aufwendiger.

Der erfindungsgemäße Ansatz dient dazu, die exakte Lage des Kontaktelementes (hier auch als Kontaktglas bezeichnet) zu bestimmen. Da die Grenzfläche des Kontaktglases i.d.R. gekrümmt ist, wird deren Form üblicherweise in einem Koordinatensystem beschrieben, das auf das Kontaktglas bezogen ist, bei einer gekrümmten Kontaktfläche beispielsweise auf einen Scheitelpunkt oder einen sonstigen ausgezeichneten Punkt der Krümmung. Die Materialbearbeitung bzw. die Vorrichtung zur Materialbearbeitung wird hingegen unter Bezug auf ein Koordinatensystems betrieben, das auf die Vorrichtung selbst referenziert ist. Die Bestimmung der exakten Lage der Kontakt- oder Eintrittsfläche des Kontaktgtases erlaubt es nun, das Koordinatensystem des Kontaktglases bzw. dessen Flächenkrümmung auf das Koordinatensystem der Vorrichtung bzw. des Materialbearbeitungsverfahrens abzugleichen bzw. abzustimmen bzw. den zwischen den beiden Koordinatensystemen bestehenden Versatz zu ermitteln und zu berücksichtigen.

Die Meßfläche wird so gewählt, daß sie die zu erwartende Lage der zu erfassenden Fläche des Kontaktelementes schneidet. Die zu erwartende Lage ist vorbekannt, da das Kontaktglas vor der Lagenbestimmung an der Bearbeitungsvorrichtung befestigt wird. Aufgrund der damit bekannten geometrischen Verhältnisse ist der Bereich, in dem die Lage der zu erfassenden Fläche erwartet wird, durch die Toleranzen bei der Befestigung sowie die möglichen Variationen, die sich bei der Fertigung des Kontaktglases oder bei Kontaktgläsern verschiedener Art ergeben können, vorgegeben.

Durch Verstellen der Fokuslage der Meßlaserstrahlung in der Meßfläche werden Schnittpunkte zwischen Meßfläche und zu erfassender Fläche gesucht. Die konfokale Detektion erlaubt es dabei, den Brechzahlsprung, der an der Fläche des vorzugsweise an dieser Stelle nicht kontaktierten Kontaktglases auftritt, klar zu detektieren. Wird der Fokus in der Meßfläche verstellt, tritt immer dann, wenn der Fokus an einem Schnittpunkt zwischen zu erfassender Fläche und Meßfläche liegt, ein Brechzahlsprung auf, der zu einem entsprechenden Signal bei der konfokalen Detektion führt.

Die konfokale Detektion der rückgestreuten oder -reflektierten Meßlaserstrahlung nutzt vorteilhaft aus, daß der Anteil der an einer Grenzfläche eines transparenten Mediums rückgestreuten Sendestrahlung, die konfokal detektiert wird, signifikant höher ist, als innerhalb des transparenten Mediums. Die konfokale Detektion liefert durch die dabei auftretende räumliche Filterung ein ausreichendes Signal, dessen Stärke im wesentlichen von der Brechzahldifferenz der an der Kontaktfläche aneinandergrenzender Medien abhängig ist. Sie ist also z.B. für den Übergang vom Glas zur Luft größer, als für den Übergang von Glas zu Tränenflüssigkeit. Es ist deshalb bei der Bestimmung der Lage der Kontaktfläche bevorzugt die Lageerfassung durchzuführen, bevor das Kontaktelement auf das zu bearbeitende Objekt, z.B. die Augenhornhaut, aufgesetzt wird. Da die Energie der Meßlaserstrahlung keinen Bearbeitungseffekt zur Folge hat, ist es natürlich auch möglich, die Lage der Fläche zu ermitteln, nachdem das Kontaktelement auf das Objekt aufgesetzt wurde, im Falle der Augenchirurgie also mit Tränenflüssigkeit benetzt ist.

Natürlich muß die Meßfläche nicht vollständig durch die Fokusverstellung angefahren werden. Es genügt völlig, wenn hinreichend viele Punkte, d.h. eine (genügend) dichte Bahnkurve in der Meßfläche liegt. Die zu erfüllende Bedingung ist, daß für die Lagebestimmung ausreichend viele Schnittpunkte gefunden werden. Notfalls muß die Punktzahl in der Meßfläche erhöht werden. Weiter genügt für die Meßfläche jede zweidimensionale Mannigfaltigkeit, deren Ausdehnung so ist, daß sie die erwartete Lage der bekannten zu erfassenden Fläche schneidet.

Es ist deshalb bevorzugt, daß die Fokuslage entlang einer Bahnkurve verstellt wird, die in der Meßfläche liegt. Die Bahnkurve sollte so gewählt werden, daß sie die gesuchte Fläche des Kontaktglases schneidet, d.h. gemeinsame Punkte zwischen Meßfläche und Fläche in deren zu erwartender Lage enthält. Im allgemeinen genügt es, wenn die Bahnkurve bei einer gekrümmten zu erfassenden Fläche mindestens fünf unterscheidbare Schnittpunkte zwischen Meßfläche und zu erfassender Fläche liefert.

Aus der Lage der Schnittpunkte wird dann unter Berücksichtigung der vorbekannten Eintritts- oder Kontaktflächenform die Lage dieser Fläche ermittelt. Das kann bei nicht-sphärischen Flächen auch beinhalten, eine Verkippung der optischen Hauptachse des Systems festzustellen.

Bei einer rotationssymmetrischen Fläche genügen dabei weniger Schnittpunkte. Für solche Flächen, die üblicherweise bei sphärisch gekrümmten Kontaktelementen verwendet werden, ist es zu bevorzugen, daß die Meßfläche zylindersymmetrisch um die optische Hauptachse des Bearbeitungslaserstrahls liegt, vorzugsweise die Form einer Zylindermantelfläche oder einer Kreisscheibe aufweist. Eine Zylindermantelfläche ist besonders günstig, da sich gezeigt hat, daß die z-Koordinate, d.h. die Koordinate längs der optischen Hauptachse des Bearbeitungslaserstrahls, hinsichtlich der Befestigung des Kontaktelementes und dessen Fertigungstoleranzen besonders großen Schwankungen unterworfen ist. Bildet man die Meßfläche als Zylindermantelfläche aus, ergibt sich der Vorteil, daß ein besonders großer Bereich in z-Richtung (längs der optischen Hauptachse der Meß- oder der Bearbeitungslaserstrahlung) abgedeckt werden kann. Die Bahnkurve in dieser Zylindermantelfläche kann beispielsweise als Spirale ausgebildet werden. Wählt man die Steigung dieser Spirale hinreichend gering, ist sichergestellt, daß eine ausreichende Anzahl an Schnittpunkten und der (rotationssymmetrischen) zu erfassenden Fläche gegeben sind. Falls erforderlich, d.h. falls nicht ausreichend (s.o.) Schnittpunkte auftreten, ist die Bahnkurve iterativ zu verändern.

Die Detektion der Fläche wird natürlich einfacher, wenn die Leistung der Meßlaserstrahlung erhöht wird. Ein optimales.Signal-Rausch-Verhältnis- erreicht man bereits bei- einer Impulsenergie von etwa 20 nJ. Die Intensität der Meßlaserstrahlung, die insbesondere gepulst aufgebracht werden kann, ist so gewählt, daß kein optischer Durchbruch im Fokus erfolgt. Bevorzugt ist bei einer gepulsten Meßlaserstrahlung deshalb eine Pulsenergie von unter 300 nJ. Dieser Wert stellt eine Obergrenze dar, die nicht überschritten werden sollte, um optische Durchbrüche oder sonstige nicht-lineare Bearbeitungseffekte am Kontaktelement auszuschließen.

Generell empfiehlt sich weiter die Anwendung möglichst geringer Meßlaserstrahlungsleistung, um die Strahlenbelastung für Anwender oder Dritte möglichst gering zu halten. Hierfür ist natürlich nicht nur die Pulsenergie, sondern auch die Pulsfrequenz relevant, da beide zusammen die Dosis festlegen. Bei einer Pulsfrequenz zwischen 50 und 500 kHz ist günstigerweise eine Obergrenze von 10 nJ, oder sogar 5 nJ, vorteilhaft, da dann bei üblichen Wellenlängen zwischen 1.000 nm und 1.060 nm die von der Augenlinse eines Betrachters maximal auf die Netzhaut fokussierbare Strahlungsleistung im unschädlichen Bereich bleibt.

Eine Bearbeitungswirkung auf ein transparentes Objekt kann durch einen optischen Durchbruch erreicht werden. Es ist aber auch möglich, einen Bearbeitungseffekt dadurch zu erreichen, daß in ein bestimmtes Volumenelement innerhalb eines bestimmten engen Zeitabstandes wiederholt Laserstrahlungspulse eingebracht werden, die unterhalb der Schwelle für einen optischen Durchbruch liegen. Diese Wirkung wird üblicherweise so erklärt, daß die Pulsenergie EPULS der Laserstrahlung innerhalb vergleichsweise kurzer Zeit im Volumen akkumuliert werden kann und so zu einem Bearbeitungseffekt führt. Um dies zu vermeiden, sollte die Anzahl an Laserimpulsen, die als Meßlaserstrahlung innerhalb von weniger als 20 Sekunden ausgesendet werden, innerhalb der maximalen Ausdehnung des Volumens eine bestimmte Anzahl nicht überschreiten. Bezeichnet man die maximale Ausdehnung der Bahnkurve mit D und die Pulsfrequenz mit f ergibt sich daraus die Ungleichung: $f < 20Hz \, ((D / E_{PULS}) * (1\mu J / 1mm))^4$. Die maximale Ausdehnung D ist dabei diejenige Ausdehnung der Bahnkurve, bei der sie einem Betrachter als leuchtendes Objekt erscheinen würde, wenn er in der Lage wäre, den Spektralbereich der Meßlaserstrahlung (mit entsprechender Ortsauflösung) wahrzunehmen. D sollte mindestens 1 μm betragen, jedoch nicht mehr als 20 mm. Diese Maße ergeben sich aus den möglichen Abweichungen der zu erwartenden Lage der gesuchten Fläche,

Die genannte Ungleichung berücksichtigt auch die nicht zu überschreitende Strahlenbefastung durch die Meßlaserstrahlung. Da die maximale Ausdehnung des Objektes in diese Ungleichung mit der vierten Potenz eingeht, kann durch geeignete Wahl der Bahnkurve, insbesondere geeignete Wahl deren lateraler Ausdehnung, erheblich reduziert werden.

Zur noch weitergehenden Sicherheit für Anwender oder Dritte kann währen der Lagebestimmung auch eine Schutzvorrichtung eingesetzt werden, die möglicherweise durch das Kontaktelement transmittierte Meßlaserstrahlung absorbiert. Beispielsweise kann eine Schutzkappe am Kontaktelement auf der der Einfallseite der Meßlaserstrahlung gegenüberliegenden Seite aufgesetzt werden, wobei zur Erhaltung eines Brechzahlsprunges an der Kontaktfläche vorzugsweise die Schutzkappe die Kontaktfläche nicht kontaktiert. Dies ist auch aus Sterilitätsgründen vorteilhaft. Alternativ oder zusätzlich kann ein Abdeckmechanismus verwendet werden, der an der Bearbeitungsvorrichtung integriert ist und aktiviert, z.B. eingeschwenkt wird.

Insbesondere bei der Laserchirurgie kommen oftmals verschiedene Kontaktelemente zum Einsatz, die sich hinsichtlich der Geometrie ihrer Eintritts- und/oder Kontaktfläche unterscheiden. Ein Unterscheidungsparameter kann beispielsweise die Krümmung der Fläche oder der Durchmesser des Kontaktelementes sein. Bei der Materialbearbeitung bzw. beim Betrieb der entsprechenden Vorrichtung ist natürlich zu berücksichtigen, welches Kontaktelement, d.h. welche Eintritts- bzw. Kontaktfläche aktuell vorliegt. Bei der Bestimmung der Lage der Fläche ist dann deren Form nicht exakt bekannt. Wohl weiß man aber, daß die Form der Fläche aus einer bestimmten Gruppe von Formen stammt, da der Vorrat an möglichen Kontaktelementen natürlich begrenzt ist. Nicht zuletzt bei rotationssymmetrischen Kontaktelementen, auf jeden Fall aber bei sphärischen Flächen gibt die gegenseitige Lage von Scheitelpunkt der Fläche und Rand der Fläche eine eindeutige Aussage darüber, welche Fläche aus der begrenzten Gruppe von Flächen das Kontaktelement aufweist, das aktuell an der Vorrichtung befestigt ist. Es ist deshalb bevorzugt, daß die Lage des Scheitelpunkts der gekrümmten Fläche wie die Lage des Randes der Fläche ermittelt und aus dem Abstand bzw. der gegenseitigen Lage dieser Strukturen festgestellt wird, welches Kontaktelement bzw. welche Kontaktfläche vorhanden ist. Diese Feststellung kann umfassen, den Krümmungsradius zu vermitteln.

Wie bereits erwähnt, wird die eingangs genannte Aufgabe auch durch eine entsprechende Vorrichtung gelöst. Insoweit vorangehend oder nachfolgend Verfahrensschritte, die bei der Vorbereitung einer solchen Vorrichtung ausgeführt werden, erläutert wurden/werden, werden solche Verfahrensschritt an der Vorrichtung automatisch gesteuert durch ein entsprechendes Steuergerät ausgeführt. Eine Mitwirkung einer behandelnden Person ist nicht erforderlich. Insbesondere kann das Verfahren vollautomatisch durch Aufruf einer entsprechenden Routine in einem Steuerprogramm der Vorrichtung ausgeführt werden. Dies gilt gleichermaßen für Varianten, bei denen das zu bearbeitende Objekt noch nicht am Kontaklelement antiegt, wie auch für Varianten, bei denen das Kontaktelement bereits auf das Objekt aufgesetzt ist. Da im übrigen die Meßlaserstrahlung keine Bearbeitungswirkung hat (siehe oben), ist das erfindungsgemäße Verfahren nicht therapeutischer oder diagnostischer Natur auch wenn es im Zusammenhang mit der Augenchirurgie eingesetzt wird.

Natürlich können die hier vorstehend oder nachfolgend geschilderten Ausgestaltungen und Merkmale auch einzeln oder in anderen nicht ausdrücklich genannten Kombinationen vorteilhaft eingesetzt werden.

Die Erfindung wird nachfolgend unter Bezugnahme auf die Zeichnung beispielhalber noch näher erläutert. Es zeigen:

Fig. 1    eine schematische Darstellung einer Vorrichtung zur Materialbearbeitung in Form einer Behandlungsvorrichtung zur Augenchirurgie,

Fig. 2    eine vergrößerte Schemadarstellung eines Kontaktglases, das in der Vorrichtung der Figur 1 verwendet wird,

Fig. 3    eine nochmals vergrößerte Darstellung des Kontaktglases der Figur 2, wobei zusätzlich die Lage einer Meßfläche eingezeichnet ist, mit Hilfe derer die Lage der Unterseite des Kontaktglases bestimmt wird,

Fig. 4    eine Schemadarstellung zur Verdeutlichung der Lagebestimmung, wobei die Schemadarstellung einer Teilansicht der Figur 3 von unten entspricht, und

Fig. 5    eine Schemadarstellung einer weiteren möglichen Meßfläche sowie einer darin liegenden Bahnkurve zur Bestimmung der Lage des Kontaktglases in der Vorrichtung der Figur 1.

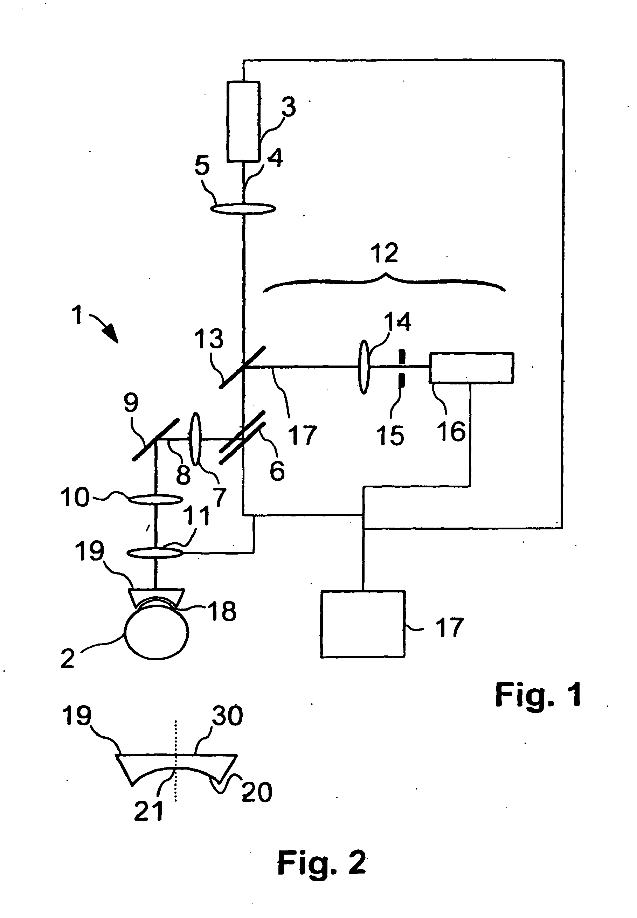

Figur 1 zeigt eine Behandlungsvorrichtung für ein augenchirurgisches Verfahren ähnlich dem in der EP 1159986 A1 bzw. der US 5549632 beschriebenen. Die Behandlungsvorrichtung 1 dient dazu, an einem Auge 2 eines Patienten eine Fehlsichtigkeitskorrektur gemäß dem bekannten LASIK-Verfahren oder einem ähnlichen Verfahren auszuführen. Dazu weist die Behandlungsvorrichtung 1 einen Behandlungs-Laser 3 auf, der gepulste Laser-Strahlung abgibt. Die Pulsdauer liegt dabei z.B. im Femtosekundenbereich, und die Laserstrahlung wirkt mittels nicht-linearer optischer Effekte in der Hornhaut auf die eingangs beschriebene Art und Weise, z.B. indem optische Durchbrüche in der Hornhaut erzeugt werden.

Der vom Laser 3 abgegebene Laserstrahl 4 fällt dabei auf einen Scanner 6, der in der beschriebenen schematischen Ausführungsform durch zwei Scanspiegel realisiert ist, welche um zueinander orthogonale Achsen drehbar sind. Der Scanner 6 fenkt den Laserstrahl zweidimensional ab. Es liegt also nach dem Scanner 6 sowie nach dessen nachgeordneter Scanoptik 7 ein Strahlfächer 8 vor, der gegenüber einer optischen Hauptachse der Einfallsrichtung abhängig von der Stellung der Scanner 6 um gewisse Winkel ausgelenkt ist. Nach Umlenkung durch einen Strahlteiler 9, der einen optischen Einblick für einen Benutzer schafft, wird der Strahtfächer durch eine Tubuslinse 10 sowie ein verstellbares Objektiv 11 in einen Fokus gebündelt, der im vorderen Abschnitt des Auges 2, z.B. der Hornhaut 18, liegt. Für jeden Strahl des Strahlfächers, d.h. für jede Stellung des Scanners 6 ist eine entsprechende laterale Verschiebung des Fokus gegenüber der optischen Hauptachse, die bei nichtausgelenkten Scannern vorliegt, realisiert.

Das verstellbare Objektiv 11 realisiert zusammen mit der Tubuslinse 10 eine Projektionsoptik, die eine Verschiebung des Fokus entlang der optischen Hauptachse, d.h. in der sogenannten z-Richtung realisiert. Die Kombination aus Objektiv 11 und Scanner 6 stellt folglich eine dreidimensional wirkende Fokusverstelleinrichtung dar. Diese Fokusverstelleinrichtung wird von einem Steuergerät 17 angesteuert, so daß damit mit der Vorrichtung 1, z.B. das bekannte LASIK-Verfahren ausgeführt werden kann.

Um, wie bereits eingangs erwähnt, die erforderlichen konstanten Einfallsverhältnisse auf die Hornhaut 18 zu erreichen und diese auch räumlich zu fixieren, ist auf die Hornhaut 18 ein Kontaktglas 19 aufgesetzt, auf das später noch eingegangen wird.

Die Behandlungsvorrichtung 1 entspricht insoweit der bekannten Bauweise, wie sie auch in der WO 2004/032810 A2 beschrieben ist. Gegenüber dem dort beschriebenen Gerät ist die Behandlungsvorrichtung 1 allerdings um einen konfokalen Detektor 12 erweitert. Der konfokale Detektor 12 ist über einen Strahlteiler 13 in den Strahlengang des einfallenden Laserstrahls 4 vor dessen Ablenkung durch den Scanner 6 eingebunden. Der strahlteiler 13 liegt also im ruhenden Strahlengang und hat die Wirkung eines aus der Laserscanningmikroskopie bekannten Farbteilers, wobei hier auch eine nicht-spektrale Teilerwirkung möglich ist.

Der konfokale Detektor 12 detektiert Strahlung, die in der Hornhaut 18, d.h. im Fokus, der durch die dreidimensional wirkende Fokusverstellvorrichtung ausgewählt ist, rückgestreute oder - reflektierte Strahlung und koppelt sie am Strahlteiler 13 aus. Die zu detektierende Strahlung durchläuft den Strahlengang des Laserstrahls 4 vom Fokus bis zum Strahlteiler 13 in entgegengesetzter Richtung.

Eine Pinholeoptik 14 sowie ein nachgeordnetes Pinhole 15 bewirken die gewünschte konfokale Filterung bezüglich des Fokus in der Hornhaut 18, so daß nur aus dem Fokus rückgestreute oder -reflektierte Strahlung zum weiter nachgeordneten Detektor 16 gelangt. Dieser ist ebenfalls über (nicht bezeichnete) Leitungen mit dem Steuergerät 17 verbunden, das unter Rückgriff auf die entsprechende Ansteuerung der dreidimensionalen Fekusverstellvorrichtung (Scanner 6 und Objektiv 11) das Signal vom Detektor 16 der jeweiligen Fokuslage zuordnen und so ein Bild erzeugen kann.

Das in der Vorrichtung 1 der Figur 1 verwendete Kontaktglas 19 ist als Schnittzeichnung in Figur 2 schematisch vergrößert dargestellt. Wie zu sehen ist, weist es plane Eintrittsfläche 30 und eine Kontaktfläche 20 auf, die im Ausführungsbeispiel rotationssymmetrisch, im allgemeinen jedoch eben oder gekrümmt ist. Wie aus der WO 2004/032810 A2 bekannt, können auch ebene Kontaktgläser verwendet werden. Auch kann die Eintrittsfläche 30 gekrümmt sein. Das in Figur 2 gezeigte rotationssymmetrische Kontaktglas 19 weist für die Kontaktfläche 20 einen Scheitelpunkt 21 auf, der bei rotationssymmetrischer Kontaktfläche 20 als Durchtrittspunkt der optischen Achse des Kontaktglases 19 durch die Kontaktfläche 20 definiert ist. Natürlich ist bei einem Kontaktglas 19 mit gekrümmter Eintrittsfläche 30 (auch) hier ein Scheitelpunkt gegeben. Nachfolgend wird jedoch exemplarisch von der Bauweise der Figur 2 ausgegangen.

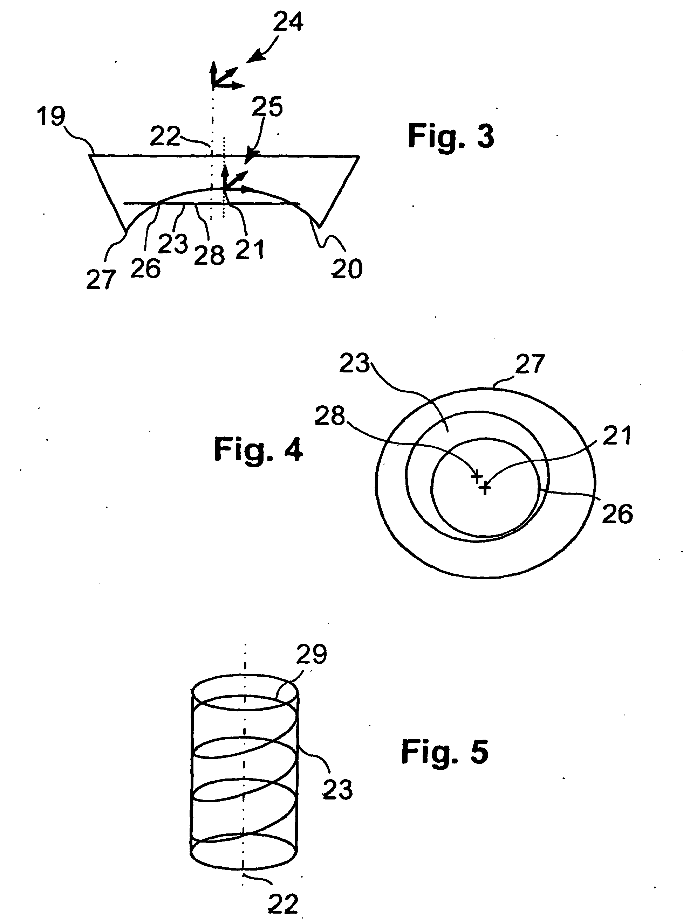

Wie Figur 3 zeigt, wird üblicherweise die Krümmung der Kontaktfläche- 20 in einem Koordinatensystem (beispielsweise in Zylinder- oder Kugelkoordinaten) beschrieben, das auf den Scheitelpunkt 21 bezogen ist. Dieses Koordinatensystem ist in Figur 3 schematisch eingezeichnet und mit dem Bezugszeichen 25 versehen.

Nach Befestigen des Kontaktglases 19 an der Behandlungsvorrichtung 1, beispielsweise mittels einer Mechanik, wie sie in der WO 05/048895 A1 beschrieben ist, hat das Kontaktglas 19 (und damit auch dessen Kontaktfläche 20) eine feste räumliche Lage zur Behandlungsvorrichtung 1, die allerdings mit Toleranz behaftet ist.

Die dreidimensionale Verstellung des Fokus erfolgt in der Behandlungsvorrichtung 1 in einem Koordinatensystem 24, das auf eines der im Betrieb vorhandenen Elemente der Behandlungsvorrichtung 1 bezogen ist, üblicherweise auf den Scanner 6 oder die Kontaktfläche des Kontaktglases. Dieses Koordinatensystem 24 ist in Figur 3 schematisch eingezeichnet. Es fällt in der Regel nicht mit dem Koordinatensystem 25 zusammen, in dem die Kontaktglaskrümmung beschrieben ist. Dies liegt daran, daß die optische Hauptachse 22 der einfallenden Laserstrahlung aufgrund unvermeidlicher Toleranzen bei der Befestigung des Kontaktglases 19 sowie aufgrund Fertigungstoleranzen für das Kontaktglas regelmäßig lateral gegenüber der optischen Achse bzw. dem Scheitelpunkt 21 des Kontaktglases 19 verschoben und/ oder verkippt sein kann. Auch liegt regelmäßig eine gewisse Unsicherheit hinsichtlich der Lage des Scheitelpunktes 21 in z-Richtung, d.h. längs der optischen Hauptachse 22 vor, da insbesondere die Mittendicke eines Kontaktglases 19 ferligungstechnisch sehr aufwendig eng zu tolerieren ist.

Zur Bestimmung des Versatzes zwischen den Koordinatensystemen 24 (der Behandlungsvorrichtung 1) und 25 (des Kontaktglases 19) wird durch den Strahlengang der Behandlungsvorrichtung 1 Meßlaserstrahlung eingestrahlt. Zweckmäßigerweise wird dabei der Behandlungslaser 3 als Strahlungsquelle für die Meßlaserstrahlung verwendet, da der Behandlungslaser 3 in der vorgestellten Ausführungsform in einem Betriebszustand gesteuert werden kann, in dem er gepulste Laserstrahlung mit einer Pulsenergie abgeben kann, die im Fokus, d.h. nach Durchlauf der Optik der Behandlungsvorrichtung 1, keine nicht-lineare Wechselwirkung, insbesondere keinen optischen Durchbruch im Fokus zur Folge hat. - Geeignete Abschwächer sind ebenfalls möglich. Natürlich kann auch eine separate Strahlungsquelle für Meßlaserstrahlung verwendet werden. Wesentlich ist allerdings, daß die Meßlaserstrahlung eine hinreichend exakte Beziehung zum Koordinatensystem 24 aufweist. Dies ist besonders einfach zu gewährleisten, wenn die Meßlaserstrahlung ebenfalls die Fokusverstellvorrichtung, d.h. den Scanner 6, die Tubuslinse 10 und das Objektiv 11 durchläuft, also im Koordinatensystem 24 der Vorrichtung verstellt wird. Nur dann kann der Versatz zwischen den beiden Koordinatensystemen hinreichend exakt bestimmt werden.

Die Meßlaserstrahlung in Form eines niederenergetischen Laserstrahls 4 wird nun entlang einer Bahnkurve verstellt, die in einer Meßfläche 23 liegt. Die Lage der Meßfläche 23 ist dabei so gewählt, daß sie die zu erwartende Lage der Kontaktfläche 20 schneidet. In der Ausführungsform, die in Figur 3 gezeigt ist, ist eine Meßfläche 23 ausgewählt, die in Zylinderkoordinaten des Koordinatensystems 24 beschrieben auf konstanter z-Koordinate liegt. Die Meßfläche 23 ist also eine Fläche senkrecht zur optischen Hauptachse 22. Die Koordinaten der Bahnkurve in der Meßfläche 23 dieser Ausführungsform unterscheiden sich also hinsichtlich ihrer radialen bzw. Winkelkoordinaten, haben jedoch eine konstante z-Koordinate. Die Schnittmenge zwischen der Meßfläche 23 und der Kontaktfläche 20 ist eine geschlossene Bahnkurve, die bei der hier vorliegenden sphärisch gekrümmten Kontaktfläche 20 kreisförmig ist, da ein nicht den Mittelpunkt enthaltener Kugelschnitt regelmäßig zu einem Kleinkreis führt. Dieser Kleinkreis ist nun gegenüber der optischen Hauptachse 22 verschoben.

Diese Verschiebung ist in Figur 4 gut zu erkennen, welche die Darstellung der Figur 3 von unten zeigt. Aus der Verschiebung sowie dem Radius des Kleinkreises, der in Form der Schnittlinie 26 in Figur 4 eingezeichnet ist, läßt sich die Verschiebung des Koordinatensystems 25 leicht berechnen. Dies gilt nicht nur für sphärische Kontaktflächen, sondern ganz allgemein für rotationssymmetrische, sofern die Form der Kontaktfläche bekannt ist. Im Falle einer sphärischen Kontaktfläche 20 sind die radialen und Winkelkoordinaten des Zentrums des Kleinkreises, auf dem die Schnittpunkte 26 liegen, automatisch die entsprechenden lateralen Koordinaten des Scheitelpunktes 21. Die z-Koordinate Za ergibt sich aus der z-Koordinate Zk der Meßfläche 23, sowie dem Krümmungsradius R der Kontaktfläche 23 und dem Radius r des erwähnten Kleinkreises durch die Gleichung: $Za = Zk + R - (R^2 - r^2)^{1/2}$.

In den Figuren 3 und 4 ist zusätzlich zu den erwähnten Parametern / Strukturen auch noch der Rand 27 der Kontaktfläche 20 eingezeichnet. Dieser Rand 27 kann bei geeigneter Verschiebung der Meßfläche 23 längs der optischen Hauptachse 22 ebenfalls detektiert werden. Man muß dazu lediglich eine Schar von Meßflächen 23 verwenden, um den Rand 27 aufzufinden. Beispielsweise aus den radialen Koordinaten von Scheitelpunkt 21 und Rand 27 kann dann auch im Fall einer Kontaktfläche 20, die aus einer Gruppe möglicher Kontaktflächen ausgewählt ist, diejenige Kontaktfläche 20 bestimmt werden, die aktuell vorliegt. Der Fachmann kann dazu beispielsweise auf dem Unterschied in Radialkoordinaten zwischen Scheitelpunkt 21 und Rand 27 zugreifen. Nach der Bestimmung, welche Kontaktfläche aus der bekannten Gruppe vorliegt, kann dann die erwähnte Abgleichung der Koordinatensysteme 25 und 24 hochpräzise erfolgen, ohne daß man zuvor genau wissen mußte, welches der Kontaktgläser 19 aus einer Gruppe möglicher Kontaktgläser tatsächlich an der Vorrichtung 1 befestigt wurde.

Figur 5 zeigt eine weitere Ausführungsform, bei der eine andere Meßfläche 23 verwendet wurde. Hier gilt für alle Punke der Meßfläche 23, daß in Zylinderkoordinaten des Koordinatensystems 24 der Radius konstant ist. Die Meßfläche 23 ist eine Zylindermanteifläche. Die in dieser Manteifläche liegende Sahnkurve kann beispielsweise als Spirale 29 ausgebildet werden. Die Schnittpunkte 26 (die zur einfacheren Darstellung von Figur 5 nicht eingezeichnet sind), die die Grenzfläche 20 mit der Meßfläche 23 hat, liegen wiederum auf einer geschlossenen Bahnkurve. Die Bestimmung der Lage der Kontaktfläche 20 ist hierbei für den Fachmann einfach möglich, insbesondere kann bei einer sphärischen Kontaktfläche eine einfache analytische Lösung angewendet werden.

## Patentansprüche

1. Verfahren zum Vorbereiten einer Vorrichtung (1) zur Materialbearbeitung durch Erzeugung optischer Durchbrüche in oder an einem Objekt (18), die eine variable, dreidimensional wirkende Fokusverstelleinrichtung (6, 11) zur Fokussierung gepulster Bearbeitungslaserstrahlung (4) auf verschiedene Orte im oder auf dem Objekt (18) aufweist, wobei

   - an der Vorrichtung ein für die Bearbeitungslaserstrahlung (4) transparentes auf das Objekt (18) aufzusetzendes Kontaktelement (19) befestigt wird, das auf seiner auf das Objekt (2) aufzusetzenden Seite eine Kontakt-Fläche (20) und eine dieser gegenüberliegende Einiritts-Fläche für die Bearbeitungslaserstrahlung aufweist, die jeweils vorbekannte Form haben,
   - vor der Bearbeitung des Objektes (18) die Lage der Eintritts- oder Kontakt-Fläche (20, 30) bezüglich der Fokusverstelleinrichtung (6, 11) mittels Einstrahlung von Laserstrahlung (4) auf die Fläche bestimmt wird, indem Meßlaserstrahlung (4) mittels der variablen Fokusverstelleinrichtung (6,11) nahe der oder auf die Fläche (20, 30) fokussiert wird, wobei die Energiedichte der fokussierten Meßlaserstrahlung (3) zur Erzeugung eines optischen Durchbruches zu gering ist, und der Fokus der Meßlaserstrahlung (4) in einer Meßfläche (23) verstellt wird, die die erwartete Lage der Fläche (20, 30) schneidet,

   **dadurch gekennzeichnet, daß**

   a) aus dem Fokus der Meßlaserstrahlung (4) rückgestreute oder -reflektierte Strahlung konfokal detektiert wird.
   b) aus der konfokal detektierten Strahlung und der zugeordneten Einstellung der variablen Fokusverstelleinrichtung (6, 11) die Lage von Schnittpunkten (26) zwischen Meßfläche (23) und Fläche (20, 30) ermittelt wird, wobei nötigenfalls der Schritt a) mit einer geänderten, insbesondere verschobenen. Meßfläche (23) mehrmals wiederholt wird, bis eine bestimmte Anzahl, vorzugsweise fünf, Schnittpunkte (26) detektiert wurde,
   c) aus der Lage der Schnittpunkte (26) und der vorbekannten Form der Fläche (20, 30) deren Lage bestimmt wird.

2. Verfahren nach Anspruch 1, **dadurch gekennzeichnet, daß** die Fläche (20. 30) nichtsphärisch ist und daß die Lage auch hinsichtlich einer Verkippung der Fläche (20) zur optischen Achse bestimmt wird.

3. Verfahren nach Anspruch 1, **dadurch gekennzeichnet, daß** die Fokuslage entlang einer Bahnkurve (28) verstellt wird, die in der Meßfläche (23) liegt:

4. Verfahren nach einem der obigen Ansprüche, **dadurch gekennzeichnet, daß** die Meßfläche (23) zylindersymmetrisch zur optischen Hauptachse (22) des Bearbeitungslaserstrahls (4) ist, vorzugsweise die Form einer Zylindermantelfläche oder einer Kreisscheibe aufweist.

5. Verfahren nach einem der obigen Ansprüche, **dadurch gekennzeichnet, daß** die Meßlaserstrahlung (4) gepulst mit einer Pulsenergie EPULS ≤300 nJ ist.

6. Verfahren nach Anspruch 5, **dadurch gekennzeichnet, daß** eine in der Meßfläche (23) liegende Bahnkurve (28) verwendet wird, die eine maximale Ausdehnung D hat und für deren Pulsfrequenz f gilt:

$$f < 20Hz \cdot ((D \,/\, EPULS) \cdot (1\mu J \,/\, 1mm))^4$$

7. Verfahren nach einem der obigen Ansprüche, **dadurch gekennzeichnet, daß** eine in der Meßfläche (23) liegende Bahnkurve (28) verwendet wird, die eine maximale Ausdehnung D hat, welche zwischen 1 $\mu$m und 15 mm liegt.

8. Verfahren nach einem der obigen Ansprüche, **dadurch gekennzeichnet, daß** die Meßlaserstrahlung (4) aus einer auch für die Erzeugung der Bearbeitungslaserstrahlung vorgesehenen, gepulsten Laser-Strahlungsquelle (3) bereitgestellt wird, indem die Strahlquelle: (3) in einen Betrieb mit verminderter Pulsenergie gesteuert wird oder ein Energieminderer im Strahlengang der Bearbeitungslaserstrahlung (4) aktiviert bzw. eingesetzt wird.

9. Verfahren nach einem der obigen Ansprüche, **dadurch gekennzeichnet, daß** die Schritte a) - c) des Anspruchs 1 ausgeführt werden, nachdem das Kontaktelement (19) gegenüber der Fokusverstelleinrichtung fixiert wurde, aber bevor das Kontaktelement (19) auf das Objekt (18) gesetzt wird.

10. Verfahren nach einem der obigen Ansprüche, **dadurch gekennzeichnet, daß** während der Einstrahlung der Meßlaserstrahlung (4) die Kontaktfläche (20) abgedeckt wird, vorzugsweise nicht-kontaktierend.

11. Verfahren nach einem der obigen Ansprüche, **dadurch gekennzeichnet, daß** bei einer gekrümmten Fläche (20. 30) die Lage eines Scheitelpunkts (21) der Fläche (20, 30) ermittelt- und als Bezugspunkt für die spätere Materialbearbeilung bereitgehalten wird.

12. Verfahren nach Anspruch 11, **dadurch gekennzeichnet, daß** die Form der Fläche (20, 30) dahingehend vorbekannt ist, daß eine Gruppe mehrerer unterschiedlicher, jeweils in ihrer exakten Geometrie bekannter Formen festlegt, aus der die Form der Fläche (20. 30) stammt, daß die Lage des Flächen-Randes (27) ermittelt wird und aus der Relativlage von Rand und Scheitelpunkt (21) diejenige Form der-Gruppe ermittelt wird, die die Fläche (20, 30) hat.

13. Materialbearbeitungs-Vorrichtung mit

  - einem Bearbeitungslaser (3), der gepulste Bearbeitungslaserstrahlung (4) bereitstellt,
  - einer Optikeinrichtung (5, 10) zum Fokussieren der Bearbeitungslaserstrahlung (4) in oder auf ein zu bearbeitendes Objekt (18) derart, daß im Fokus optische Durchbrüche entstehen,
  - einer Fokusverstelleinrichtung (6, 11) zum variablen Verstellender Fokuslage im oder auf dem Objekt (18),
  - einem an der Vorrichtung (1) befestigbares Kontaktelement (19) zum Aufsetzen auf das Objekt (18), das eine auf das Objekt (18) aufzusetzende Kontakt-Fläche (20) und eine dieser gegenüberliegende Eintritts-Fläche für die Bearbeitungslaserstrahlung aufweist, die jeweils vorbekannte Form haben, und
  - einer Steuereinrichtung (17) zur Bestimmung der Lage der Eintritts- oder Kontakt-Fläche (20, 30) nach der Befestigung des Kontaklelementes (19) und vor der Bearbeitung des Objektes (18), die den Bearbeitungslaser (3) und die Fokusverstelleinrichtung (6, 11) ansteuert, wobei
  - eine ebenfalls von der Steuereinrichtung (17) angesteuerte Meßlaserstrahlungsquelle (3) zur Abgabe von Meßlaserstrahlung (4) vorgesehen ist, deren Meßlaserstrahlung (4) die Fokusverstelleinrichtung (6, 11) und die Optikeinrichtung (5, 10) durchläuft und im Fokus keine optischen Durchbrüche bewirkt, wobei die Steuereinrichtung (17) zur Bestimmung der Lage der Fläche (20, 30) den Fokus der Meßlaserstrahlung (4) in einer Meßfläche (23) verstellt, welche die zu erwartende Lage der Fläche (20, 30) schneidet,

**dadurch gekennzeichnet, daß**

  - eine konfokate Detektoreinrichtung (12) vorgesehen ist, die aus dem Fokus der Meßlaserstrahlung (4) rückgestreute oder -reflektierte Strahlung konfokal detektiert und Meßsignale an die Steuereinrichtung (17) liefert, und
  - die Steuereinrichtung (17) so ausgebildet ist, dass sie aus den Meßsignalen die Lage von Schnittpunkten (26) zwischen Meßfläche (23) und Fläche (20, 30) ermittelt, wobei die Steuereinrichtung (17) nötigenfalls die Meßfläche variiert, insbesondere verschiebt, falls keine oder zu wenige Schnittpunkte auftreten, und die aus der Lage der Schnittpunkte (26) und der vorbekannten Form der Fläche (20, 30) deren Lage (19) bestimmt.

**14.** Vorrichtung nach Anspruch 13, **dadurch gekennzeichnet, daß** die Steuereinrichtung (17) die Vorrichtung (1) zur Ausführung eines der Verfahren gemäß einem oder mehreren der obigen Verfahrensansprüche steuert.

**15.** Vorrichtung nach einem der obigen Vorrichtungsansprüche, **gekennzeichnet durch** einen das Kontaktelement (19) an der Kontaktfläche (20) abdeckenden Abdeckmechanismus zur Absorption transmittierter Meßlaserstrahtung (4).

**16.** Vorrichtung nach einem der obigen Vorrichlungsansprüche, **dadurch gekennzeichnet, daß** die Meßlaserstrahlung (4) gepulst ist und die Pulsenergie zwischen 2 nJ und 300 nJ liegt.

**17.** Vorrichtung nach einem der obigen Vorrichtungsansprüche, **dadurch gekennzeichnet, daß** die Meßlaserstrahlungsquelle durch die in einen Betriebsmodus zur Abgabe niederenergelischer Laserstrahlungspulse gesteuerte Bearbeitungslaserstrahlungsquelle (3) realisiert ist.

**Claims**

**1.** A method of preparing an apparatus (1) for material processing by producing optical breakthroughs in or on an object (18), said apparatus comprising a variable three-dimensionally acting focus adjustment device (6, 11) for focusing pulsed processing laser radiation (4) on different locations in or on the object (18), wherein

- a contact element (19), which is transparent for the processing laser radiation (4) and is to be placed on the object (18), is mounted to the apparatus, said contact element (19) having, on its side to be placed on the object (18), a contact surface (20) and, opposite said contact surface (20), an entry surface (30) for the processing laser radiation, each of said surfaces having a known shape;
- prior to processing the object (18), the position of the entry surface (30) or contact surface (20) with respect to the focus adjustment device (6, 11) is determined by irradiation of laser radiation (4) on said surface, by focusing measurement laser radiation (4) near or on said surface (20, 30) by means of the variable focus adjustment device (6, 11), the energy density of the focused measurement laser radiation (3) being too low to produce an optical breakthrough, and the focus of the measurement laser radiation (4) being adjusted in a measurement surface (23) which intersects the expected position of said surface (20, 30),

**characterized in that**

a) radiation scattered back or reflected back from the focus of the measurement laser radiation (4) is confocally detected,
b) the position of points of intersection (26) between the measurement surface (23) and said surface (20, 30) is determined from the confocally detected radiation and the assigned setting of the variable focus adjustment device (6, 11), with step a) being repeated several times, if necessary, with a modified, in particular shifted, measurement surface (23) until a determined number of points of intersection (26), preferably five, have been detected,
c) the position of said surface is determined from the position of the points of intersection (26) and the previously known shape of said surface (20, 30).

**2.** The method as claimed in claim 1, **characterized in that** said surface (20, 30) is non-spherical and that the position is determined also with respect to a tilt of said surface (20) relative to the optical axis.

**3.** The method as claimed in claim 1, **characterized in that** the focus position is shifted along a path curve (28) which is located in the measurement surface (23).

**4.** The method as claimed in any one of the above claims, **characterized in that** the measurement surface (23) is cylindrically symmetrical to the main optical axis (22) of the processing laser beam (4) and preferably has the shape of a cylinder barrel or of a circular disk.

**5.** The method as claimed in any one of the above claims, **characterized in that** the measurement laser radiation (4) is pulsed with a pulse energy EPULS $\leq$ 300 nJ.

**6.** The method as claimed in claim 5, **characterized in that** a path curve (28) is used which is located in the measurement surface (23) and which has a maximum extent D and a pulse frequency f for which

$$f < 20Hz * ((D / EPULS) * (1\mu J / 1mm))^4$$

holds true.

7. The method as claimed in any one of the above claims, **characterized in that** a path curve (28) is used which is located in the measurement surface (23) and which has a maximum extent D of between 1 $\mu$m and 15 mm.

8. The method as claimed in any one of the above claims, **characterized in that** the measurement laser radiation (4) is provided by a pulsed laser radiation source (3), which is also intended to generate the processing laser radiation, by controlling the radiation source (3) in a mode of reduced pulse energy or by activating or inserting an energy attenuator in the beam path of the processing laser radiation (4).

9. The method as claimed in any one of the above claims, **characterized in that** steps a) - c) of claim 1 are carried out after the contact element (19) has been fixed with respect to the focus adjustment device, but before the contact element (19) is placed on the object (18).

10. The method as claimed in any one of the above claims, **characterized in that** the contact surface (20) is covered, preferably in a contactless manner, during irradiation of the measurement laser radiation (4).

11. The method as claimed in any one of the above claims, **characterized in that**, in case of a curved surface (20, 30) the position of a vertex (21) of the surface (20, 30) is determined and is stored as a reference point for the subsequent material processing.

12. The method as claimed in claim 11, **characterized in that** the shape of the surface (20, 30) is previously known to belong to a group of several different shapes, each known with respect to its exact geometry, wherein further the position of a surface edge (27) is determined and, based on the relative position of the edge and of a vertex (21), that shape from said group of shapes which the surface (20, 30) has is determined.

13. A material processing apparatus, comprising

- a processing laser (3) which provides pulsed processing laser radiation (4),
- an optical device (5, 10) for focusing the processing laser radiation (4) in or on an object (18) to be processed such that optical breakthroughs form in the focus,
- a focus adjustment device (6, 11) for variable adjustment of the focus position in or on the object (18),
- a contact element (19) to be mounted to the apparatus (1), which contact element (19) is to be placed on the object (18) and comprises a contact surface (20) to be placed on the object (18) and, opposite said contact surface (20), an entry surface for the processing laser radiation, each of said surfaces having a previously known shape, and
- a control device (17) for determining the position of the entry surface (30) or contact surface (20) after mounting of the contact element (19) and before processing of the object (18), said control device (17) controlling the processing laser (3) and the focus adjustment device (6, 11), wherein
- a measurement laser radiation source (3), likewise controlled by the control device (17), is provided to emit measurement laser radiation (4), the measurement laser radiation (4) from said radiation source (3) passing through the focus adjustment device (6, 11) and through the optical device (5, 10) and causing no optical breakthroughs in the focus, wherein the control device (17), in order to determine the position of the surface (20, 30), shifts the focus of the measurement laser radiation (4) in a measurement surface intersecting the expected position of the surface (20, 30),

**characterized in that**

- a confocal detector device (12) is provided which confocally detects radiation scattered back or reflected back from the focus of the measurement laser radiation (4) and supplies measurement signals to the control device (17), and
- the control device (17) determines the position of points of intersection (26) between the measurement surface (23) and the surface (20, 30) on the basis of the measurement signals, said control device (17) varying, in particular shifting, the measurement surface, if necessary, in case there are no or too few points of intersection, and determining, on the basis of the position of the points of intersection (26) and the previously known shape of the surface (20, 30), the position (19) of said surface (20, 30).

14. The apparatus as claimed in claim 13, **characterized in that** the control device (17) controls the apparatus (1) for carrying out one of the methods according to one or more of the above method claims.

15. The apparatus as claimed in any one of the above apparatus claims, **characterized by** a covering mechanism which covers the contact element (19) at the contact surface (20) for absorption of transmitted measurement laser radiation (4).

16. The apparatus as claimed in any one of the above apparatus claims, **characterized in that** the measurement laser radiation (4) is pulsed and the pulse energy is between 2 nJ and 300 nJ.

17. The apparatus as claimed in any one of the above apparatus claims, **characterized in that** the measurement laser radiation source is realized by the processing laser radiation source (3) being controlled in a mode of operation for emission of low-energy laser radiation pulses.

## Revendications

1. Procédé pour préparer un dispositif (1) à un usinage de matériau par la production d'interruptions optiques dans ou sur un objet (18), ledit dispositif présentant un système d'ajustement de foyer variable (6, 11) à effet tridimensionnel pour focaliser un rayonnement laser d'usinage pulsé (4) sur différents endroits dans ou sur l'objet (18), dans lequel

   - un élément de contact (19) transparent au rayonnement laser d'usinage (4) et à poser sur l'objet (18) est fixé au dispositif, ledit élément présentant sur son côté à poser sur l'objet (2) une surface de contact (20) et une surface d'entrée opposée à celle-ci pour le rayonnement laser d'usinage, lesdites surfaces ayant respectivement une forme préalablement connue,
   - avant l'usinage de l'objet (18), la position de la surface d'entrée ou de la surface de contact (20, 30) par rapport au système d'ajustement de foyer (6, 11) est déterminée au moyen d'une irradiation d'un rayonnement laser (4) sur la surface, en ce qu'un rayonnement laser de mesure (4) est focalisé au moyen du système d'ajustement de foyer variable (6, 11) à proximité de ou sur la surface (20, 30), la lumination du rayonnement laser de mesure (3) focalisé étant trop faible pour produire une interruption optique, et le foyer du rayonnement laser de mesure (4) étant ajusté dans une surface de mesure (23) coupant la position attendue de la surface (20, 30),

   **caractérisé en ce que**

   a) un rayonnement rétrodiffusé ou réfléchi à partir du foyer du rayonnement laser de mesure (4) est détecté de manière confocale,
   b) à partir du rayonnement détecté de manière confocale et du réglage attribué du système d'ajustement de foyer variable (6, 11), la position de points d'intersection (26) entre la surface de mesure (23) et la surface (20, 30) est déterminée, l'étape a) étant répétée à plusieurs reprises, si nécessaire, avec une surface de mesure (23) modifiée, en particulier déplacée, jusqu'à ce qu'un certain nombre de points d'intersection (26), de préférence cinq, aient été détectés,
   c) à partir de la position des points d'intersection (26) et de la forme préalablement connue de la surface (20, 30), la position de celle-ci est déterminée.

2. Procédé selon la revendication 1, **caractérisé en ce que** la surface (20, 30) n'est pas sphérique et **en ce que** la position est également déterminée concernant un défaut d'alignement angulaire de la surface (20) par rapport à l'axe optique.

3. Procédé selon la revendication 1, **caractérisé en ce que** la position de foyer est ajustée le long d'une trajectoire (28) qui est située dans la surface de mesure (23).

4. Procédé selon l'une quelconque des revendications précédentes, **caractérisé en ce que** la surface de mesure (23) est cylindrosymétrique à l'axe optique principal (22) du rayon laser d'usinage (4), présentant de préférence la forme d'une surface latérale de cylindre ou d'un disque circulaire.

5. Procédé selon l'une quelconque des revendications précédentes, **caractérisé en ce que** le rayonnement laser de mesure (4) est pulsé avec une énergie d'impulsion EPULS ≤ 300 nJ.

6. Procédé selon la revendication 5, **caractérisé en ce qu'**une trajectoire (28) située dans la surface de mesure (23) est utilisée qui présente une extension maximale D et pour la fréquence d'impulsion f de laquelle :

$$f < 20 \text{ Hz} * ((D/EPULS) * (1 \text{ } \mu J / 1 \text{ mm}))^4$$

s'applique.

7. Procédé selon l'une quelconque des revendications précédentes, **caractérisé en ce qu'**une trajectoire (28) située dans la surface de mesure (23) est utilisée qui présente une extension maximale D située entre 1 $\mu$m et 15 mm.

8. Procédé selon l'une quelconque des revendications précédentes, **caractérisé en ce que** le rayonnement laser de mesure (4) est fourni à partir d'une source de rayonnement laser pulsé (3), prévue pour la production du rayonnement laser d'usinage, **en ce que** la source de rayon (3) est commandée dans un mode à énergie d'impulsion réduite, ou un réducteur d'énergie est activé ou mis en oeuvre sur la trajectoire des rayons du rayonnement laser d'usinage (4).

9. Procédé selon l'une quelconque des revendications précédentes, **caractérisé en ce que** les étapes a) à c) de la revendication 1 sont exécutées après que l'élément de contact (19) a été fixé par rapport au système d'ajustement de foyer, mais avant que l'élément de contact (19) n'ait été posé sur l'objet (18).

10. Procédé selon l'une quelconque des revendications précédentes, **caractérisé en ce que** pendant l'irradiation du rayonnement laser de mesure (4), la surface de contact (20) est couverte, de préférence sans contact.

11. Procédé selon l'une quelconque des revendications précédentes, **caractérisé en ce que** pour une surface courbe (20, 30), la position d'un sommet (21) de la surface (20, 30) est déterminée et réservée comme point de référence pour l'usinage de matériau ultérieur.

12. Procédé selon la revendication 11, **caractérisé en ce que** la forme de la surface (20, 30) est préalablement connue dans la mesure où un groupe de plusieurs formes différentes, respectivement connues quant à leur géométrie exacte, est défini dont est issue la forme de la surface (20, 30), **en ce que** la position du bord de surface (27) est déterminée et à partir de la position relative du bord et du sommet (21) celle des formes du groupe est déterminée qui présente la surface (20, 30).

13. Dispositif d'usinage de matériau, comprenant :

- un laser d'usinage (3) fournissant un rayonnement laser d'usinage pulsé (4),
- un système optique (5, 10) pour focaliser le rayonnement laser d'usinage (4) dans ou sur un objet (18) à usiner de telle sorte que des interruptions optiques se créent au niveau du foyer,
- un système d'ajustement de foyer (6, 11) pour l'ajustement variable de la position de foyer dans ou sur l'objet (18),
- un élément de contact (19) pouvant se fixer au dispositif (1), destiné à être posé sur l'objet (18) et présentant une surface de contact (20) à poser sur l'objet (18) et une surface d'entrée, opposée à celle-ci, pour le rayonnement laser d'usinage, lesdites surfaces présentant respectivement des formes préalablement connues, et
- un système de commande (17) pour déterminer la position de la surface d'entrée ou de la surface de contact (20, 30) après la fixation de l'élément de contact (19) et avant l'usinage de l'objet (18), ledit système de commande pilotant le laser d'usinage (3) et le système d'ajustement de foyer (6, 11), dans lequel
- une source de rayonnement laser de mesure (3) également pilotée par le système de commande (17) est prévue pour délivrer un rayonnement laser de mesure (4) et dont le rayonnement laser de mesure (4) passe par le système d'ajustement de foyer (6, 11) et le système optique (5, 10) et ne provoque pas d'interruptions optiques au niveau du foyer, le système de commande (17) pour déterminer la position de la surface (20, 30) ajustant le foyer du rayonnement laser de mesure (4) dans une surface de mesure (23) coupant la position attendue de la surface (20, 30),

**caractérisé en ce que**

- un système de détection confocale (12) est prévu qui détecte de manière confocale un rayonnement rétrodiffusé ou réfléchi à partir du foyer du rayonnement laser de mesure (4) et fournit des signaux de mesure au système de commande (17), et

- le système de commande (17) est réalisé de telle sorte qu'il détermine à partir des signaux de mesure la position de points d'intersection (26) entre la surface de mesure (23) et la surface (20, 30), dans lequel le système de commande (17) fait varier, en particulier déplace, la surface de mesure si nécessaire, si aucun point d'intersection n'apparaît ou si trop peu de points d'intersection apparaissent, et qui détermine à partir de la position des points d'intersection (26) et de la forme préalablement connue de la surface (20, 30) la position (19) de celle-ci.

14. Dispositif selon la revendication 13, **caractérisé en ce que** le système de commande (17) commande le dispositif (1) pour exécuter l'un des procédés selon une ou plusieurs des revendications de procédé précédentes.

15. Dispositif selon l'une quelconque des revendications de dispositif précédentes, **caractérisé par** un mécanisme de recouvrement recouvrant l'élément de contact (19) au niveau de la surface de contact (20) en vue de l'absorption du rayonnement laser de mesure (4) transmis.

16. Dispositif selon l'une quelconque des revendications de dispositif précédentes, **caractérisé en ce que** le rayonnement laser de mesure (4) est pulsé et l'énergie d'impulsion se situe entre 2 nJ et 300 nJ.

17. Dispositif selon l'une quelconque des revendications de dispositif précédentes, **caractérisé en ce que** la source de rayonnement laser de mesure est réalisée par la source de rayonnement laser d'usinage (3) commandée dans un mode de fonctionnement pour délivrer des impulsions de rayonnement laser à faible énergie.

**Fig. 1**

**Fig. 2**

**Fig. 3**

**Fig. 4**

**Fig. 5**

IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6373571 B
- EP 1159986 A2
- WO 03002008 A1
- DE 19831674 A1
- WO 05039462 A1
- DE 10354025 A1
- WO 04032810 A2
- WO 040328810 A2
- EP 1159986 A1
- US 5549632 A
- WO 2004032810 A2
- WO 05048895 A1